# EUROPÄISCHE PATENTSCHRIFT

(11) **EP 0 856 513 B1**
(45) Veröffentlichungstag und Bekanntmachung des Hinweises auf die Patenterteilung: **30.11.2005**
(21) Anmeldenummer: 98100259.5
(22) Anmeldetag: 09.01.1998
(51) Int. Cl.: C07D 401/04, A61K 31/445

(54) **Thalidomidanaloge Verbindungen aus der Klasse der Piperidin-2,6-Dione**
Thalidomide analogues from the piperidine-2,6-dione class
Analogues de la thalidomide de la classe des pipéridine-2,6-diones

(30) Priorität: 01.02.1997 DE 19703763
(43) Veröffentlichungstag der Anmeldung: 05.08.1998
(73) Patentinhaber: Grünenthal GmbH, 52078 Aachen (DE)
(72) Erfinder: Zimmer, Oswald, Dr., 52146 Würselen (DE); Winter, Werner, Prof. Dr., 52078 Aachen (DE); Wnendt, Stephan, Dr., 52062 Aachen (DE); Zwingenberger, Kai, Dr., 52076 Aachen (DE); Eger, Kurt, Prof. Dr., 72072 Tübingen (DE); Teubert, Uwe, 04758 Oschatz (DE)

(56) Entgegenhaltungen:
- WO-A-92/14455
- WO-A-98/03502
- DE-A- 4 211 812
- US-A- 5 463 063
- US-A- 5 698 579
- CHEMICAL ABSTRACTS, vol. 66, no. 21, 22.Mai 1967 Columbus, Ohio, US; abstract no. 94788a, G. CASINI ET AL.: "Reactions of a stable primary enamine, 2-amino-4-phenyl-4-ethylglutaconimide" Seite 8861; XP002072714 & ANN. CHIM. (ROME), Bd. 56, Nr. 12, 1966, Seiten 1519-1530,
- U. TEUBERT ET AL.: "5'-Substituted thalidomide analogs as modulators of TNF-alpha" ARCH. PHARM. (WEINHEIM, GER.), Bd. 331, Nr. 1, 1998, Seiten 7-12, XP002072713
- FERNANDEZ L.P. ET AL: 'Does thalidomide affect IL-2 response and production?' EXPERIMENTAL HEMATOLOGY Bd. 23, 1995, Seiten 978 - 985

## Beschreibung

Die Erfindung betrifft thalidomidanaloge Verbindungen aus der Klasse der Piperidin-2,6-Dione, ein Verfahren zu deren Herstellung sowie ihre Verwendung in Arzneimitteln.

Die überschießende Bildung des Zytokins TNF-α (Tumornekrosefaktor α) spielt eine zentrale Rolle in der Pathogenese des Graft-versus-Host-Syndroms, der Multiplen Sklerose, der Transplantatabstoßung, aphthöser Stomatitis, Erythema nodosum leprosum, Morbus Boeck, rheumatoider Arthritis und einer Reihe weiterer Erkrankungen, die mit entzündlichen Erscheinungen einhergehen. Ein Ansatz für die Therapie dieser Erkrankungen besteht in der gezielten Unterdrückung der TNF-a-Freisetzung durch Gabe immunsupprimierender oder -modulierender Wirkstoffe, wie zum Beispiel Dexamethason, Pentoxifyllin oder Thalidomid.

Allerdings muß zwischen Indikationen unterschieden werden, die eine generelle Immunsuppression erfordern und solchen, bei denen Vor- und Nachteile einer Immunsuppression abzuwägen sind. Thalidomid hat sich in der Behandlung der aphthösen Stomatitis im Vergleich zu den klassischen Immunsuppressiva als überlegen herausgestellt. Weitere Beispiele von Erkrankungen, in denen Thalidomid eine gute Wirksamkeit zeigte, ohne zu einer generellen Immunsuppression zu führen, sind kutaner Lupus erythematodes (H+G 69, 816 bis 822 (1994)), Pyoderma gangränosum und orogenitale Ulcera bei Morbus Behcet (The Lancet, 20.05.89, 1093 bis 1095). Als pathogenetische Faktoren dieser auf Haut und Schleimhäute beschränkten Läsionen gelten endogene Mediatoren mit Wirkungen auf das Endothel und zirkulierende Leukozyten. Unter dem Einfluß von TNF-α und anderer Zytokine nimmt fokal die Adhäsivität des Endothels gegenüber Leukozyten zu, dies trägt maßgeblich zur Ausbildung der Vaskulitiden bei. Bei systemischen Krankheitsbildern ist die Wirkung des Thalidomids selbst auf die Haut und Schleimhäute beschränkt, was (zusätzliche) Immunsuppression erfordert. Beispiele hierfür sind der systemische Lupus erythematodes, der neben den Hauterscheinungen auch lebensbedrohliche Veränderungen innerer Gefäße, insbesonders der Niere, aufweist; Leprareaktion vom Typ II mit Beteiligung der Augen und/oder der Gelenke sowie Morbus Behcet mit Beteiligung der Augen und/oder der Gelenke.

Substanzen, die wie Thalidomid diese Veränderung des Endothels unterdrücken, zugleich aber Reaktionen der spezifischen zellulären Immunabwehr ganz oder teilweise blockieren können einen bedeutenden Fortschritt für die Therapie der genannten systemischen Krankheitsbilder darstellen. Ein Schlüsselbotenstoff der zellulären Immunantwort ist das Interleukin 2, von dem die Proliferation antigenspezifischer Lymphozyten abhängt.

Bei der Entwicklung neuer Arzneimittel ist es deshalb ein Ziel, die anti-inflammatorischen Eigenschaften des Thalidomids gemeinsam mit immunsuppressiven Wirkkomponenten zum Ausdruck zu bringen, die Thalidomid allein in seiner klinischen Anwendung nicht hat.

Die der Erfindung zugrundeliegende Aufgabe bestand in der Entwicklung thalidomidanaloger Verbindungen aus der Klasse der Piperidin-2,6-dione, die die entzündlich ausgelöste Freisetzung von TNF-α sowie die antigeninduzierte Synthese von Interleukin 2 hemmen.

Es wurde gefunden, daß die erfindungsgemäßen Verbindungen die gestellten Anforderungen erfüllen.

Gegenstand der Erfindung sind deshalb in Position 3 und 5 substituierte Piperidin-2,6-dione der allgemeinen Formel (I) 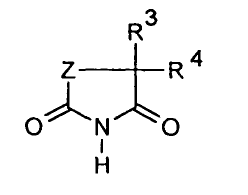 worin Z für eine der Gruppen 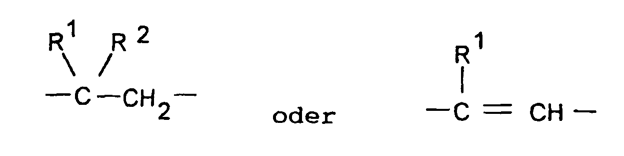 wobei das Kohlenstoffatom mit dem Substituenten R¹ an die Carbonylgruppe gebunden ist und
in der R¹ den Phthalimidrest bedeutet (wenn Z = -C(R¹R²)-CH₂- ist) oder für einen einfach oder doppelt mit Hydroxy-, Methoxy- oder Aminogruppen substituierten Phthalimidrest steht (wenn Z -C(R¹) = CH- darstellt),
R² Wasserstoff oder C₁₋₆-Alkyl (geradkettig oder verzweigt) ist,
R³ für Wasserstoff, eine C₁₋₆-Alkylgruppe (geradkettig oder verzweigt) oder ein aromatisches oder heteroaromatisches Ringsystem steht, und R⁴ eine C₁₋₆-Alkylgruppe (geradkettig oder verzweigt) oder ein aromatisches oder heteroaromatisches Ringsystem bedeutet.

Von den Piperidin-2,6-dionen der Formel (I), in der Z = -C(R¹R²)-CH₂-R¹ Phthalimid und R² und R³ Wasserstoff bedeuten, eignet sich insbesondere die Verbindung in der R⁴ Phenyl ist.

Von den Piperidin-2,6-dionen der Formel I, in der Z = -C(R¹)=CH- ist und R³ Ethyl und R⁴ Phenyl bedeuten, eignet sich insbesondere die Verbindung in der R¹ 3,4-Dimethoxyphthalimid ist.

Weiterer Erfindungsgegenstand ist ein Verfahren zur Herstellung der thalidomidanalogen Verbindungen aus der Klasse der Piperidin-2,6-Dione der allgemeinen Formel (I).

Verbindungen der allgemeinen Formel (I) mit Z = -C(R¹R²)-CH₂- lassen sich durch Kondensation von Phthalsäureanhydrid mit einer substituierten Glutaminsäure wie z.B. 4-Phenylglutaminsäure oder 4-Methylglutaminsäure in einem organischen Lösungsmittel, vorzugsweise Pyridin, Cyclisierung des Produkts in Acetanhydrid und nachfolgende Überführung in das Imid herstellen. Die Umwandlung des Anhydrids in das Imid erfolgt dabei durch Schmelzen mit Harnstoff.

Diese Zielverbindungen der Formel (I) lassen sich auch durch Umsetzung von Phthalsäureanhydrid mit einem 5-substituierten 3-Amino-glutarimid, vorzugsweise durch Erhitzen in Essigsäure, erhalten.

Verbindungen der allgemeinen Formel (I) mit Z = -C(R¹)=CH- lassen sich durch Kondensation eines substituierten Phthalsäureanhydrids wie z.B. 3,4-Dimethoxyphthalsäureanhydrid mit 5-substituierten 3-Amino-3,4-dehydropiperidin-2,6-dionen wie z.B. 3-Amino-5-ethyl-5-phenyl-glutaconimid in einem organischen Lösungsmittel, beispielsweise Essigsäure, darstellen.

### Beispiel 1

### 2-(5-Methyl-2,6-dioxo-piperidin-3-yl)-1,3-dihydro-2H-isoindol-1,3-dion(1)

2,00 g (11 mmol) 4-Methylglutaminsäure und 1,95 g (13 mmol) Phthalsäureanhydrid wurden in 15 ml trockenem Pyridin 6 h zum Rückfluß erhitzt. Nach destillativer Entfernung des Lösungsmittels wurde der Rückstand in 10 ml Acetanhydrid 1 h zum Sieden erhitzt. Der beim Abkühlen ausgefallene Feststoff wurde abgesaugt und das Filtrat eingeengt. Nach Versetzen des Filtrats mit Ether wurde der gebildete Niederschlag abgesaugt und die vereinigten Niederschläge aus absolutem Toluol umkristallisiert. 2,00 g (7 mmol) des Kristallisats und 0,23 g (3,8 mmol) Harnstoff wurden gut gemischt und im Ölbad bei ca. 200°C für 30 min. geschmolzen. Die erstarrte Schmelze wurde nacheinander mit 4 ml Acetanhydrid und 6 ml Ethanol kurz zum Sieden erhitzt. Der ausgefallene Feststoff wurde abgesaugt und aus DMF/Wasser umkristallisiert. Es wurden 1,35 g (67 % der Theorie) 2-(5-Methyl-2,6-dioxo-piperidin-3-yl)-1,3-dihydro-2H-isoindol-1,3-dion(1) mit einem Schmelzpunkt von 270 bis 272°C erhalten.

### Beispiel 2

### 2-(5-Phenyl-2,6-dioxo-piperidin-3-yl)-1,3-dihydro-2H-isoindol-1,3-dion(2)

3,00 g (12 mmol) 4-Phenylglutaminsäure und 2,12 g (14 mmol) Phthalsäureanhydrid wurden in 40 ml trockenem Pyridin 6 h zum Rückfluß erhitzt. Nach destillativer Entfernung des Lösungsmittels wurde der Rückstand in 50 ml 5 %iger HCl aufgenommen und mit Ethylacetat extrahiert. Die organische Phase wurde mit Wasser gewaschen, mit Aktivkohle entfärbt und über Natriumsulfat getrocknet. Nach destillativer Entfernung des Lösungsmittels wurde der Rückstand in 40 ml Acetanhydrid 1 h zum Rückfluß erhitzt. Anschließend wurde die Lösung eingeengt und mit Ether versetzt. Der ausgefallene Niederschlag wurde abgesaugt und aus trockenem Toluol umkristallisiert. 2,00 g (6 mmol) des Kristallisats und 0,19 g (3 mmol) Harnstoff wurden im Ölbad bei ca. 200°C für 30 min. geschmolzen. Die erstarrte Schmelze wurde nacheinander mit 4 ml Acetanhydrid und 8 ml Ethanol kurz zum Sieden erhitzt. Der ausgefallene Feststoff wurde aus DMF/Wasser umkristallisiert. Es wurden 0,80 g (40 % der Theorie) 2-(5-Phenyl-2,6-dioxo-piperidin-3-yl)-1,3-dihydro-2H-isoindol-1,3-dion(2) mit einem Schmelzpunkt von 228 bis 231°C erhalten.

### Beispiel 3

### 2-(5-Ethyl-5-phenyl-2,6-dioxo-piperidin-3-yl)-1,3-dihydro-2H-isoindol-1,3-dion(3)

1,00 g (4 mmol) 3-Amino-5-ethyl-5-phenyl-glutaconimid wurden in 40 ml wasserfreiem Ethanol gelöst, die Lösung mit 0,1 g Palladiumkohle (10 % Pd/C) versetzt und unter einer Wasserstoffatmosphäre 8,5 h gerührt. Anschließend wurde vom Katalysator filtriert und das Filtrat zur Trockne eingeengt. Der Rückstand wurde mit 0,70 g (5 mmol) Phthalsäureanhydrid in 40 ml Eisessig zum 4 h Rückfluß erhitzt. Nach destillativer Entfernung des Lösungsmittels wurde der Rückstand aus Ethanol umkristallisiert. Es wurden 0,99 g (63 % der Theorie) 2-(5-Ethyl-5-phenyl-2,6-dioxo-piperidin-3-yl)-1,3-dihydro-2H-isoindol-1,3-dion(3) mit einem Schmelzpunkt von 174 - 177°C erhalten.

### Beispiel 4

### 2-(5-Ethyl-5-phenyl-2,6-dioxo-1,2,5,6-tetrahydropyridin-3-yl)-4,5-dimethoxy-1,3-dihydro-2H-isoindol-1,3-dion(4)

0,45 g (2 mmol) 3-Amino-5-ethyl-5-phenyl-glutaconimid und 0,45 g (2 mmol) 4,5-Dimethoxyphthalsäureanhydrid wurden in 15 ml Eisessig 5 h zum Rückfluß erhitzt. Anschließend wurde zur Trockne eingeengt und der Rückstand aus Ethanol umkristallisiert. Es wurden 0,55 g (67 % der Theorie) 2-(5-Ethyl-5-phenyl-2,6-dioxo-1,2,5,6-tetrahydropyridin-3-yl)-4,5-dimethoxy-1,3-dihydro-2H-isoindol-1,3-dion(4) mit einem Schmelzpunkt von 203 - 205°C erhalten.

Die erfindungsgemäßen Verbindungen sind toxikologisch unbedenklich und eignen sich deshalb als pharmazeutische Wirkstoffe. Dementsprechend ist Erfindungsgegenstand auch die Verwendung der thalidomidanalogen Verbindungen aus der Klasse der Piperidin-2,6-Dione der Formel (I) als Wirkstoffe in Arzneimitteln, vorzugsweise als Suppressoren für die entzündlich ausgelöste Freisetzung von TNF-α sowie die antigen induzierte Synthese von Interleukin-2.

Erfindungsgemäße Arzneimittel enthalten neben mindestens einer Verbindung der allgemeinen Formel (I) Trägermaterialien, Füllstoffe, Lösungsmittel, Verdünnungsmittel, Farbstoffe und/oder Bindemittel. Die Auswahl der Hilfsstoffe sowie die einzusetzenden Mengen hängen davon ab, ob das Arzneimittel oral, intravenös, intraperitoneal, intradermal, intramuskulär, intranasal, buccal oder lokal appliziert werden soll. Für die orale Applikation eignen sich Zubereitungen in Form von Tabletten, Kautabletten, Dragees, Kapseln, Granulaten, Tropfen, Säften oder Sirupen, für die parentale, topische und inhalative Applikation Lösungen, Suspensionen, leicht rekonstituierbare Trockenzubereitungen sowie Sprays. Erfindungsgemäße Verbindungen in einem Depot in gelöster Form, einer Trägerfolie oder einem Pflaster, gegebenenfalls unter Zusatz von die Hautpenetration fördernden Mitteln, sind Beispiele für geeignete perkutane Applikationsformen. Aus oral oder perkutan anwendbaren Zubereitungsformen können die erfindungsgemäßen Verbindungen verzögert freigesetzt werden.

Die an Patienten zu verabreichende Wirkstoffmenge variiert in Abhängigkeit vom Gewicht des Patienten, von der Applikationsart, der Indikation und dem Schweregrad der Erkrankung. Üblicherweise werden 1 bis 150 mg/kg, wenigstens einer thalidomidanalogen Verbindung der Formel (I) appliziert.

### Pharmakologische Untersuchungen

Die Freisetzung von TNF-α kann in vitro an humanen mononuklearen Zellen des peripheren Blutes (T-Zellen, B-Zellen und Monozyten) nach Stimulation mit Lipopolysaccharid (LPS) untersucht werden. LPS ist ein Bestandteil der bakteriellen Zellwand und stimuliert Monozyten und Makraphagen.

Neben der Stimulation mit LPS kann die Freisetzung von TNF-α auch durch Stimulation von humanen mononukleären Zellen des peripheren Blutes mit T-Zellen spezifischen, monoklonalen Antikörpern gegen Aktivierungsantigene (AntiCD2-AntiCD28) oder dem bakteriellen Superantigen toxisch Schock Syndrom Toxin-1 TSST-1 provoziert werden. Abgesehen von der TNF-α Freisetzung führen diese Stimulanzien unter anderem zur Bildung von Interleukin-2 (Il-2).

### LPS-Stimulation mononukleärer Zellen: Wirkung auf TNF-α

Die Freisetzung von TNF-α kann in vitro an humanen mononukleären Zellen des peripheren Blutes, also T-Zellen, B-Zellen und Monozyten nach Stimulation mit Lipopolysaccharid (LPS) untersucht werden. LPS ist ein Bestandteil der bakteriellen Zellwand und stimuliert Monozyten und Makrophagen.

Mononukleäre Zellen wurden aus dem heparinisierten Blut von mindestens drei freiwilligen Spendern gewonnen. Hierzu wurden je 20 ml Blut nach bekannten Methoden über einen Ficoll-Paque-Gradienten aufgetrennt, die Zellen wurden geerntet und dreifach mit einem Zellkulturmedium gewaschen. Dieses Zellkulturmedium bestand aus RPMI 1640 Medium, supplementiert mit 2 mM Glutamin (Life Technologies, Eggenstein), 10 % fötalem Kälberserum (Life Technologies), 50 µg/ml Streptomycin (Sigma, Deisenhofen), 50 IU/ml Penicillin (Sigma) und 100 µM β-Mercaptoethanol (Merck, Darmstadt). Die mononukleären Zellen wurden schließlich in 15 ml Zellkulturmedium aufgenommen und in 1 ml Ansätzen in sterilen 24-Loch-Inkubationsplatten (Sigma) aufgeteilt. Den 1-ml-Ansätzen wurde jeweils 1 µl Dimethylsulfoxid (DMSO, Merck) oder 1 µl einer Lösung der Testsubstanz (in DMSO; Endkonzentration im Test: 0,5; 5; 12,5 und 50 µg/ml) zugesetzt und die Ansätze wurden für eine Stunde im CO₂-Brutschrank (5 % CO₂, 90 % Luftfeuchtigkeit) inkubiert. Anschließend wurden, mit Ausnahme der Kontrollen, jeweils 2,5 µg LPS (von E. coli 0127: B8, Sigma) zugefügt. Die Inkubation der Kulturen wurde für 20 h fortgesetzt. Die Konzentration von TNF-α in den Zellkulturüberständen wurde im Anschluß an die Inkubation mit kommerziellen ELISA-Tests (Boehringer Mannheim) bestimmt. Aus den Meßwerten der nicht wirkstoffbehandelten Kontrollansätze und den mit den Testverbindungen inkubierten Ansätzen wurde die Stärke der TNF-α-Hemmung berechnet. Mit Hilfe einer Regressionsgerade wurden die Konzentrationen errechnet, die zu einer 50 %igen Hemmung der TNF-α-Freisetzung führten (IC50-Werte).

In Tabelle 1 ist der inhibitorische Einfluß der erfindungsgemäßen Verbindungen auf die LPS-induzierte Freisetzung von TNF-α dargestellt:

**Tabelle 1**

| Beispiel Nr. | Hemmung der TNF-α Freisetzung_in (%) bei einer Endkonzentration von 50 µg/ml im Test | IC₅₀ [µg/ml] |
|---|---|---|
| 1 | 48 % | n.b. |
| 2 | 69 % | 8,7 |
| 3 | 80 % | 11,0 |
| 4 | 90 % | 2,0 |

### Stimulation von T-Zellen: Hemmung von I1-2

Die Freisetzung von Interleukin-2 kann durch in vitro Stimulation humaner mononukleären Zellen des peripheren Blutes, das neben T-Zellen auch B-Zellen und Monozyten enthält, untersucht werden. Durch die polyklonale Stimulation über konstante Epitope des T-Zell-Rezeptors oder sogenannte akzessorische signalübertragende Oberflächenmoleküle erhält man eine ausgeprägtere Meßstrekke als durch die Antigenstimulation kleiner T-Zellpopulationen. Es wurde die Kombination 2 solcher akzessorischer Signale, nämlich die über die Oberflächenmoleküle CD2 und CD28 verwendet.

Mononukleäre Zellen werden aus dem heparinisierten Blut von mindestens drei freiwilligen Spendern gewonnen. Hierzu werden je 20 ml Blut nach bekannten Methoden über einen Ficoll-Paque-Gradienten aufgetrennt, die Zellen wurden geerntet und dreifach mit einem Zellkulturmedium gewaschen. Dieses Zellkulturmedium besteht aus RPMI 1640 Medium, supplementiert mit 2 mM Glutamin (Life Technologies, Eggenstein), 10 % fötalem Kälberserum (Life Technologies), 50 µg/ml Streptomycin (Sigma, Deisenhofen), 50 IU/ml Penicillin (Sigma) und 100 µM β-Mercaptoethanol (Merck, Darmstadt). Die mononukleären Zellen wurden schließlich in 15 ml Zellkulturmedium aufgenommen und in 1 ml Ansätzen in sterilen 24-Loch-Inkubationsplatten (Sigma) aufgeteilt. Den 1-ml-Ansätzen wurd jeweils 1 µl Dimethylsulfoxid (DMSO, Merck) oder 1 µl einer Lösung der Testsubstanz (in DMSO; Endkonzentration im Test: 0,5; 5; 12,5 und 50 µg/ml) zugesetzt und die Ansätze wurden für eine Stunde im CO₂-Brutschrank (5 % CO₂, 90 % Luftfeuchtigkeit) inkubiert. Anschließend wurden, mit Ausnahme der Kontrollen, jeweils 0,1 µg/ml der monoklonalen Antikörper zugesetzt (Klon-Nr. AICD2.M1 von Prof. Dr. Meuer; anti-CD28 von CLB, Amsterdam) zugefügt. Die Inkubation der Kulturen wurde für 20 h fortgesetzt. Die Konzentration von Il-2 in den Zellkulturüberständen wurde im Anschluß an die Inkubation mit kommerziellen ELISA-Tests (Boehringer Mannheim) bestimmt. Aus den Meßwerten der nicht wirkstoffbehandelten Kontrollansätze und den mit den Testverbindungen inkubierten Ansätzen wurde die Stärke der Il-2-Hemmung berechnet.

Die Substanz des Beispiels 4 hemmte unter diesen Bedingungen bei einer Konzentration von 50 µg/ml die CD2/CD28-stimulierte Synthese von Il-2 um 86 ±6 %. Bei Verwendung des Staphylokkoken-Superantigens (von E.coli 0127: B8; Sigma, Deisenhofen) TSST-1 (0,1 µg/ml) als T-Zell-Stimulus wurde die Il-2-Synthese um 77 ± 20% gehemmt.

Die vorstehend gemachten Untersuchungen zeigen, daß die thalidomidanalogen Verbindungen aus der Klasse der Piperidin-2,6-Dione der Formel (I) sowohl die entzündlich ausgesetzte Freisetzung von TNF-α sowie die antigen induzierte Synthese von Interleukin-2 hemmen.

## Patentansprüche

1. Substituierte Piperidin-2,6-dione der Formel (I), 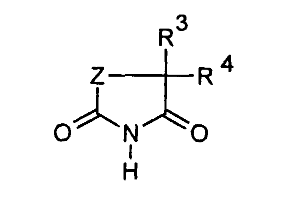 in der Z -C(R¹R²)-CH₂- oder
-C(R¹) = CH- bedeutet,
R¹ Phthalimid (wenn Z = -C(R¹R²)-CH₂-) oder einen einfach oder doppelt mit Hydroxy-, Methoxy- oder Aminogruppen substituierten Phthalimidrest (wenn Z = -C(R¹)=CH-) darstellt,
R² Wasserstoff oder ein C₁₋₆-Alkylgruppe bedeutet,
R³ Wasserstoff, eine C₁₋₆-Alkylgruppe oder ein (hetero)aromatisches Ringsystem ist und
R⁴ C₁₋₆-Alkyl oder einen (hetero)aromatischen Ring darstellt.

2. Substituierte Piperdin-2,6-dione der Formel (I) gemäß Anspruch 1, **dadurch gekennzeichnet, daß**
Z -C(R¹R²)-CH₂- ist,
R¹ Phthalimid bedeutet,
R² Wasserstoff darstellt,
R³ Wasserstoff oder Ethyl bedeutet und
R⁴ Methyl oder Phenyl darstellt.

3. Substitierte Piperidin-2,6-dione der Formel I gemäß Anspruch 1, **dadurch gekennzeichnet, daß**
Z -C(R¹)=CH- ist,
R¹ 3,4-Dimethoxyphthalimid bedeutet,
R³ Ethyl und
R⁴ Phenyl darstellt.

4. Verfahren zur Herstellung substituierter Piperidin-2,6-dione der Formel (I) gemäß Anspruch 1 und 2, **dadurch gekennzeichnet, daß** man ein Phthalsäureanhydrid mit einer substituierten Glutaminsäure kondensiert, das Produkt zum Anhydrid cyclisiert und dieses in das Imid überführt wird.

5. Verfahren zur Herstellung substituierter Piperidin-2,6-dione der Formel I gemäß Anspruch 1 bis 3, **dadurch gekennzeichnet, daß** ein Phthalsäureanhydrid mit substituierten 3-Aminoglutaconimiden oder 5-substituierten 3-Aminoglutarimiden kondensiert wird.

6. Verwendung eines substituierten Piperidin-2,6-dions der Formel I gemäß Anspruch 1 bis 3 als Wirkstoff in einem Arzneimittel.

7. Verwendung nach Anspruch 6, **dadurch gekennzeichnet, daß** das Arzneimittel ein Immunmodulator ist.

## Claims

1. Substituted piperidine-2,6-diones of formula (I) 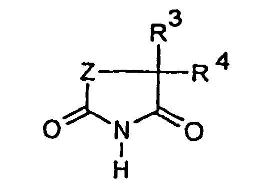 wherein Z represents -C(R¹R²)-CH₂- or -C(R¹)=CH-,
R¹ represents phthalimide (when Z = -C(R¹R²)-CH₂-) or a phthalimide radical mono- or di-substituted by hydroxy, methoxy or amino groups (when Z = -C(R¹)=CH-),
R² represents hydrogen or a C₁₋₆-alkyl group,
R³ is hydrogen, a C₁₋₆-alkyl group or a (hetero)aromatic ring system, and
R⁴ represents C₁₋₆-alkyl or a (hetero)aromatic ring.

2. Substituted piperidine-2,6-diones of formula (I) according to claim 1, **characterised in that**
Z is -C(R¹R²)-CH₂-,
R¹ represents phthalimide,
R² represents hydrogen,
R³ represents hydrogen or ethyl, and
R⁴ represents methyl or phenyl.

3. Substituted piperidine-2,6-diones of formula I according to claim 1, **characterised in that**
Z is -C(R¹)=CH-,
R¹ represents 3,4-dimethoxyphthalimide,
R³ represents ethyl, and
R⁴ represents phenyl.

4. Process for the preparation of substituted piperidine-2,6-diones of formula (I) according to claims 1 and 2, **characterised in that** a phthalic anhydride is condensed with a substituted glutamic acid, the product is cyclised to form the anhydride, and the anhydride is converted into the imide.

5. Process for the preparation of substituted piperidine-2,6-diones of formula I according to claims 1 to 3, **characterised in that** a phthalic anhydride is condensed with substituted 3-amino-glutaconimides or with 5-substituted 3-amino-glutarimides.

6. Use of a substituted piperidine-2,6-dione of formula I according to claims 1 to 3 as an active ingredient in a medicament.

7. Use according to claim 6, **characterised in that** the active ingredient is an immunomodulator.

## Revendications

1. Pipéridine-2,6-diones substituées de formule (I) 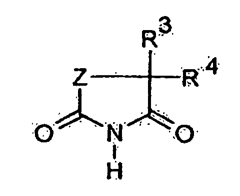 dans laquelle Z est un groupe -C(R¹R²)-CH₂- ou -C(R¹)=CH-,
R¹ est un reste phtalimide (lorsque Z représente -C(R¹R²)-CH₂-) ou un reste phtalimide substitué une ou deux fois avec des groupes hydroxy, méthoxy ou amino (lorsque Z est un groupe -C(R¹) =CH-),
R² représente l'hydrogène ou un reste alkyle en C₁ à C₆,
R³ représente l'hydrogène, un groupe alkyle en C₁ à C₆ ou un système cyclique (hétéro)aromatique,
et
R⁴ est un groupe alkyle en C₁ à C₆ ou un noyau (hétéro)aromatique.

2. Pipéridine-2,6-diones substituées de formule (I) suivant la revendication 1, **caractérisées en ce que**
Z est un groupe -C(R¹R²)-CH₂-,
R¹ est un reste phtalimide,
R² représente l'hydrogène,
R³ représente l'hydrogène ou un groupe éthyle et
R⁴ est un groupe méthyle ou phényle.

3. Pipéridine-2,6-diones substituées de formule (I) suivant la revendication 1, **caractérisées en ce que**
Z est un groupe -C(R¹)=CH-,
R¹ est un reste 3,4-diméthoxyphtalimide,
R³ est un groupe éthyle et
R⁴ est un groupe phényle.

4. Procédé de production de pipéridine-2,6-diones substituées de formule (I) suivant revendication 1 et 2, **caractérisé en ce que** l'on condense un anhydride d'acide phtalique avec un acide glutamique substitué, on cyclise le produit en anhydride et on transforme ce dernier en imide.

5. Procédé de production de pipéridine-2,6-diones substituées de formule (I) suivant revendication 1 à 3, **caractérisé en ce que** l'on condense un anhydride d'acide phtalique avec des 3-amino-glutaconimides substitués ou avec des 3-aminoglutarimides substitués en position 5.

6. Utilisation d'une pipéridine-2,6-dione substituée de formule (I) suivant revendication 1 à 3 comme substance active dans un médicament.

7. Utilisation suivant la revendication 6, **caractérisée en ce que** le médicament est un immunomodulateur.
